Europäisches Patentamt

European Patent Office

Office européen des brevets

(19)

(11) Veröffentlichungsnummer: **0 111 062**
**B1**

(12) **EUROPÄISCHE PATENTSCHRIFT**

(45) Veröffentlichungstag der Patentschrift :
**12.11.86**

(21) Anmeldenummer : **83108850.5**

(22) Anmeldetag : **08.09.83**

(51) Int. Cl.⁴ : **A 61 F 2/30**, A 61 L 2/26,
**B 65 D 75/58**

(54) Sterilisierbare Doppelverpackung.

(30) Priorität : **01.12.82 CH 6975/82**

(43) Veröffentlichungstag der Anmeldung :
**20.06.84 Patentblatt 84/25**

(45) Bekanntmachung des Hinweises auf die Patenterteilung : **12.11.86 Patentblatt 86/46**

(84) Benannte Vertragsstaaten :
**AT DE FR GB IT**

(56) Entgegenhaltungen :
**FR-A- 2 176 245
FR-A- 2 405 872
GB-A- 333 657
US-A- 2 129 887
Prospekt: Howmedica EVDB 1078351**

(73) Patentinhaber : **GEBRÜDER SULZER AKTIENGESELL-
SCHAFT
Zürcherstrasse 9
CH-8401 Winterthur (CH)**

(72) Erfinder : **Frey, Otto
Wallrütistrasse 56
CH-8400 Winterthur (CH)**
Erfinder : **Koch, Rudolf
Oberdorfstrasse 229
CH-8267 Berlingen (CH)**

(74) Vertreter : **Dipl.-Ing. H. Marsch Dipl.-Ing. K. Sparing
Dipl.-Phys.Dr. W.H. Röhl Patentanwälte
Rethelstrasse 123
D-4000 Düsseldorf (DE)**

EP 0 111 062 B1

Jouve, 18, rue St-Denis, 75001 Paris, France

## Beschreibung

Die Erfindung betrifft eine sterilisierbare Doppelverpackung für eine Endoprothese, insbesondere mit einem stielartigen Verankerungsschaft, bestehend aus zwei mit seitlichem Abstand ineinander gestapelten, trogartigen Behältern, die mit je einem Abreissdeckel verschlossen sind und von denen der innere mit Auflagern für eine Hohllagerung der Endoprothese versehen ist (Prospekt « Howmedica » EVDB 1 078 351).

Implantierbare Endoprothesen gelangen steril in einem geschlossenen Behälter auf den Operationstisch, wobei an die Sterilität bekanntlich besonders hohe Anforderungen gestellt werden. Sie werden daher vom Hersteller und Verpacker im allgemeinen in der geschlossenen Packung vorzugsweise durch Gamma-Strahlen sterilisiert. Häufig werden sie darüberhinaus unmittelbar vor dem Gebrauch beim Benutzer nochmals nachsterilisiert, wobei auch Dampf-, Gas- oder eine Formalinsterilisation angewandt werden.

Dieser hohe Aufwand ist jedoch nutzlos, wenn an der Nahtstelle zwischen sterilem und unsterilem Bereich während der Operation eine « Verunreinigung » des Implantats erfolgt. Denn bei einer Operation wird die Packung des Prothese im allgemeinen von einer unsterilen Person geöffnet und einer sterilen Person weitergegeben, die sie dem Operateur zur Entnahme des Implantats darbietet. Bei bisherigen Verpackungen der genannten Art bereitet diese Weitergabe häufig Schwierigkeiten, durch die die Gefahr eines Sterilitätsverlusts stark erhöht wird.

Aufgabe der Erfindung ist es daher, eine Verpackung zu schaffen, deren steriler Teil so dargeboten werden kann, dass die Gefahr einer Aufhebung der Sterilität durch Kontakte zwischen sterilen und unsterilen Operationshelfern vermindert wird.

Gemäss der vorliegenden Erfindung wird diese Aufgabe dadurch gelöst, dass in Längsrichtung der Behälter nach einer Seite aus der Mitte versetzt in dem äusseren Behälter ein Zugband vorgesehen ist, das den inneren Behälter aussen umgibt und mit einem Ende an dem äusseren Behälter befestigt ist.

Mit Hilfe des Zugbandes, dessen äusseres Ende vorteilhafterweise bei geschlossenem äusseren Behälter unter elastischer Vorspannung abgewinkelt zwischen den beiden Abreissdeckeln untergebracht ist, kann eine unsterile Hilfsperson — nach Entfernen des äusseren Abreissdeckels — den sterilen inneren Behälter teilweise aus dem äusseren Behälter herausheben und der sterilen Person — wie auf einem Tablett — weitergeben. Diese ergreift den sterilen Behälter in dem aus dem äusseren Behälter vorstehenden Bereich zur weiteren « Verarbeitung ». Die Möglichkeiten für Streifberührungen zwischen sterilem und unsterilem Helfer werden auf diese Weise zumindest stark eingeschränkt, wenn nicht ganz ausgeschlossen.

Die geschilderten Manipulationen lassen sich mit erhöhter Sicherheit durchführen, wenn das Zugband den inneren Behälter mindestens annähernd in der Nähe des schwersten Teils der Prothese umschliesst, da auf diese Weise die Unterstützung des inneren Behälters im wesentlichen im Bereich seines Schwerpunktes erfolgt.

Behälter und Zugband sind vorzugsweise aus Kunststoff hergestellt, wobei die Behälter beispielsweise als Spritzguss- oder Pressteile gefertigt werden. Bei der Auswahl der Kunststoffe wird neben anderen Gesichtspunkten vor allem auf ihre Sterilisierbarkeit mit Hilfe der eingangs erwähnten Verfahren geachtet. Als vorteilhafte Materialien haben sich unter Berücksichtigung weiterer Kriterien, wie z. B. Bruchfestigkeit, Polysulfone, Polymethylpenten oder Polyäthersulfon herausgestellt. Die Abreissdeckel, die vorzugsweise aus einem flanschartigen oberen Rand der Behälter aufgeschweisst oder aufgeklebt werden, bestehen zweckmässigerweise aus Spezialpapieren — z. B. Sterilisationspapier (80 g) nach DIN 5895.3, Teil 2 und 6 —, durch die hindurch eine Gas- oder Dampfdiffusion zur Sterilisierung möglich ist, die aber von Mikroorganismen und Keimen nicht « durchwandert » werden können.

Im folgenden wird die Erfindung anhand eines Ausführungsbeispiels im Zusammenhang mit der Zeichnung näher erläutert.

Figur 1 ist schematisch eine senkrecht zur Längsachse gesehene Seitenansicht einer geschlossenen Doppelpackung ;

Figur 2 zeigt eine Ansicht von Fig. 1 von der rechten Seite ;

Figur 3 gibt in gleicher Darstellung wie Fig. 2 die Doppelpackung mit geöffnetem äusseren Behälter wieder, während

Figur 4 schliesslich schematisch den mit dem « gezogenen » Zugband herausgehobenen Teil des inneren Behälters zeigt.

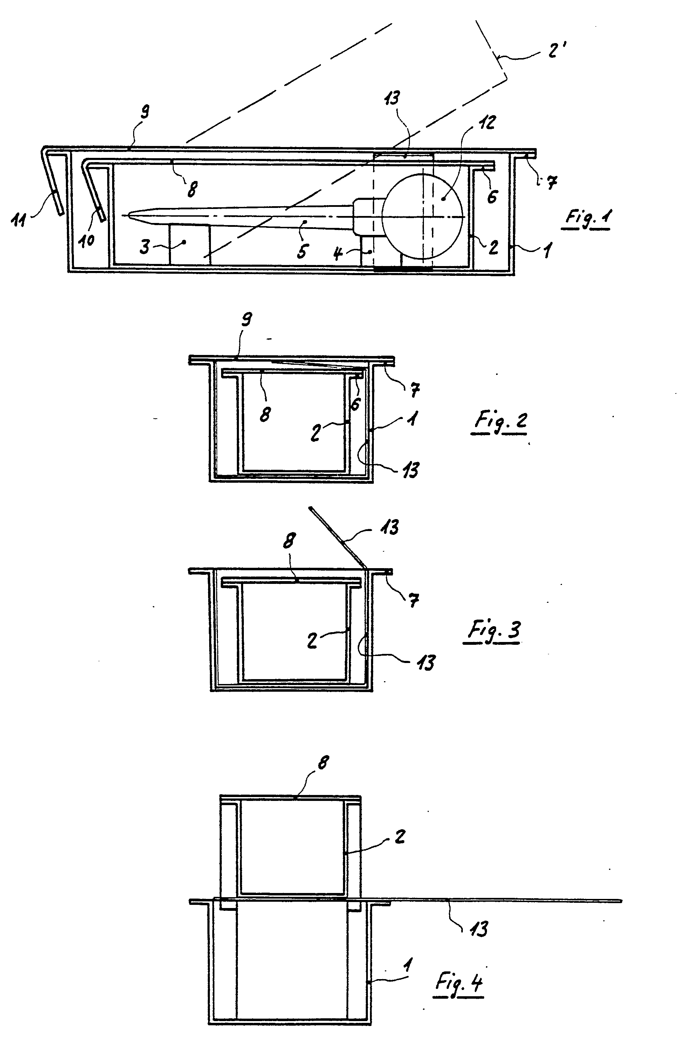

In einem trogartigen äusseren Behälter 1 (Fig. 1) ist ein gleichartiger innerer Behälter 2 mit allseitig im Spiel derart gestapelt, dass er einseitig aus dem äusseren Behälter oder Gefäss 1 in die in Fig. 1 gestrichelt eingetragene Lage 2′ gehoben werden kann.

Der innere Behälter 2 enthält Abstützt- oder Lagerelemente 3 und 4, auf denen ein Implantat 5, im vorliegenden Beispiel eine Hüftgelenkprothese, hohl gelagert werden kann. Beide Behälter 1 und 2 haben an ihrer Oberseite einen flanschartigen Rand 6 und 7, mit denen je ein mit einer Zuglasche 10 und 11 versehener Abreissdeckel 8 und 9 verbunden ist. Die vorzugsweise durch Schweissen oder Kleben hergestellte Verbindung ist mindestens keimdicht, im allgemeinen jedoch gas- oder dampfdicht ausgebildet.

Im Bereich des Gelenkkopfes 12 der Prothese 5 ist der innere Behälter 2 aussen von einem Zugband 13 umgeben, das auf einer Seite, im Rand 7 des äusseren Gefässes 1 versenkt, befestigt ist (Fig. 2) ; sein freies Ende liegt unter elastischer Vorspannung zwischen beiden Abreissdeckeln 8 und 9, so dass es nach Entfernen des Deckels 9 nach oben herausspringt (Fig. 3).

Mit der neuen Doppelverpackung erfolgt die Entnahme der Prothese beim Uebergang zwischen einer unsterilen und einer sterilen Person während einer Operation beispielsweise auf folgende Weise :

Eine unsterile Person hält den Behälter 1 und entfernt den Abreissdeckel 9. Durch die Vorspannung springt das freie Ende des Zugbandes 13 etwas nach oben vor. Die unsterile Person zieht an diesem Ende, ohne dabei den Behälter 2 und/oder den Abreissdeckel 8 zu berühren. Bei gestrecktem Zugband (Fig. 4) steht der Behälter 2 schräg im Behälter 1, wie der mit 2' bezeichnete Umriss in Fig. 1 erkennen lässt. Der Behälter 2 wird nun von einer sterilen Person entnommen ; diese reisst den Abreissdeckel 8 weg und bietet den geöffneten Behälter 2 dem Operateur zur direkten Entnahme der hohlgelagerten Prothese 5 dar.

## Patentansprüche

1. Sterilisierbare Doppelverpackung für eine Endoprothese, insbesondere mit einem stielartigen Verankerungsschaft, bestehend aus zwei mit seitlichem Abstand ineinander gestapelten trogartigen Behältern (1, 2), die mit je einem Abreissdeckel (8, 9) verschlossen sind, und von denen der innere mit Auflagern (3, 4) für eine Hohllagerung der Endoprothese (5, 12) versehen ist, dadurch gekennzeichnet, dass in Längsrichtung der Behälter (1, 2) nach einer Seite aus der Mitte versetzt in dem äusseren Behälter (1) ein Zugband (13) vorgesehen ist, das den inneren Behälter (2) aussen umgibt und mit einem Ende an dem äusseren Behälter (1) befestigt ist.

2. Doppelverpackung nach Anspruch 1, dadurch gekennzeichnet, dass das freie Ende des Zugbandes (13) bei geschlossenem äusseren Behälter (1) unter elastischer Vorspannung abgewinkelt zwischen den beiden Abreissdeckeln (8, 9) untergebracht ist.

3. Doppelverpackung nach Anspruch 1 oder 2, dadurch gekennzeichnet, dass das Zugband (13) den inneren Behälter (2) mindestens annähernd in der Nähe des schwersten Teils der Endoprothese (5, 12) umschliesst.

## Claims

1. A sterilizable double packing for an endoprosthesis, the same having more particularly a rod-like anchorage stem, the double packing comprising two trough-like receptacles (1, 2) stacked one in another with a lateral spacing, the receptacles each being closed by a tear-off cover (8, 9), the inner receptacle having supports (3, 4) for a hollow mounting of the endoprosthesis (5, 12), characterised in that a pull strip (13) which is offset lengthwise of the receptacles (1, 2) to one side from the centre is provided in the outer receptacle (1), extends around the outside of the inner receptacle (2) and has one end secured to the outer receptacle (1).

2. A double packing according to Claim 1, characterised in that with the outer receptacle (1) closed, the free end of the pull strip (13) is received bent and with resilient prestressing between the two tear-off covers (8, 9).

3. A double packing according to Claim 1 or 2, characterised in that the pull strip (13) extends around the inner receptacle (2) at least substantially in the vicinity of the heaviest part of the endoprosthesis (5, 12).

## Revendications

1. Emballage double stérilisable pour une endoprothèse présentant notamment une tige d'ancrage de configuration longiligne, comprenant deux réceptacles (1, 2) en forme d'auges qui sont empilés l'un dans l'autre avec espacement latéral, sont obturés chacun par un couvercle arrachable (8, 9), et parmi lesquels le réceptacle interne est muni d'appuis (3, 4) pour un support creux de l'endoprothèse (5, 12), caractérisé par le fait qu'il est prévu dans le réceptacle externe (1), dans le sens longitudinal des réceptacles (1, 2), une bande de traction (13) excentrée d'un côté, qui entoure extérieurement le réceptacle interne (2) et est fixée par une extrémité au réceptacle externe (1).

2. Emballage double selon la revendication 1, caractérisé par le fait que l'extrémité libre de la bande de traction (13) est intercalée entre les deux couvercles arrachables (8, 9), lorsque le réceptacle externe (1) est fermé, en étant coudée sous une précharge élastique.

3. Emballage double selon la revendication 1 ou 2, caractérisé par le fait que la bande de traction (13) entoure le réceptacle interne (2) au moins approximativement au voisinage de la partie la plus lourde de l'endoprothèse (5, 12).

_Fig. 1_

_Fig. 2_

_Fig. 3_

_Fig. 4_